# EUROPEAN PATENT SPECIFICATION

(11) **EP 1 996 113 B1**
(45) Date of publication and mention of the grant of the patent: **30.08.2017**
(21) Application number: 07733978.6
(22) Date of filing: 14.03.2007
(51) Int. Cl.: A61F 2/88, A61L 31/02, A61F 2/07

(54) **HYBRID AMORPHOUS METAL ALLOY STENT**
HYBRIDER STENT AUS AMORPHER METALLLEGIERUNG
STENT EN ALLIAGE MÉTALLIQUE AMORPHE HYBRIDE

(30) Priority: 15.03.2006 US 377769
(43) Date of publication of application: 03.12.2008
(73) Proprietor: Medinol Ltd., Tel Aviv 6158101 (IL)
(72) Inventor: RICHTER, Jacob, 47226 Ramat Hasharon (IL)
(74) Representative: Kuhnen & Wacker
(86) International application number: PCT/IB2007/000632
(87) International publication number: WO 2007/105088

(56) References cited:
- WO-A1-00/32138
- WO-A1-83/00997
- WO-A2-2004/045454
- US-A- 5 421 919
- US-A- 5 788 626
- US-A1- 2002 120 327
- US-A1- 2003 208 260
- US-A1- 2004 267 349
- US-A1- 2004 267 349
- US-A1- 2006 246 210

## Description

### FIELD OF THE INVENTION

The invention relates generally to stents, which are intraluminal endoprosthesis devices implanted into vessels within the body, such as a blood vessels, to support and hold open the vessels, or to secure and support other endoprostheses in vessels.

### BACKGROUND OF THE INVENTION

Various stents are known in the art. Typically, stents are generally tubular in shape, and are expandable from a relatively small, unexpanded diameter to a larger, expanded diameter. For implantation, the stent is typically mounted on the end of a catheter with the stent being held on the catheter at its relatively small, unexpanded diameter. Using a catheter, the unexpanded stent is directed through the lumen to the intended implantation site. Once the stent is at the intended implantation site, it is expanded, typically either by an internal force, for example by inflating a balloon on the inside of the stent, or by allowing the stent to self-expand, for example by removing a sleeve from around a self-expanding stent, allowing the stent to expand outwardly. In either case, the expanded stent resists the tendency of the vessel to narrow, thereby maintaining the vessel's patency.

Some examples of patents relating to stents include U.S. Patent No. 4,733,665 to Palmaz; U.S. Patent No. 4,800,882 and 5,282,824 to Gianturco; U.S. Patent Nos. 4,856,516 and 5,116,365 to Hillstead; U.S. Patent Nos. 4,886,062 and 4,969,458 to Wiktor; U.S. Patent No. 5,019,090 to Pinchuk; U.S. Patent No. 5,102,417 to Palmaz and Schatz; U.S. Patent No. 5,104,404 to Wolff; U.S. Patent No. 5,161,547 to Tower; U.S. Patent No. 5,383,892 to Cardon et al.; U.S. Patent No. 5,449,373 to Pinchasik et al.; and U.S. Patent No. 5,733,303 to Israel et al.

Materials used to make both permanent and removable temporary devices often must be made of strong materials which are capable of deforming or bending in accordance with the pressures and movements of the patient's body or the organ in which they are implanted. Current metals have limited fatigue resistance and some suffer from sensitivity to *in vivo* oxidation. Also, because of the fabrication methods used, many metal devices do not have acceptably smooth, uniform surfaces. This property is important to prevent an adverse response of the device in the body, and to prevent accelerated corrosion of the implanted device. Thus, it is desirable to produce these medical devices with a new material, i.e., one that is non-corrosive, highly elastic, and strong.

Stents may be constructed from flat metal, which is rolled and welded to form the tubular structure of the stent. In one such embodiment, the flat metal is in the form of a panel which is simply rolled straight and connected.

Another type of flat metal stent construction is known as the helical or coiled stent. Such a stent design is described in, for example, U.S. Patent nos. 6,503,270 and 6,355,059. This stent design is configured as a coiled stent in which the coil is formed from a wound strip of cells wherein the sides of the cells are serpentine. Other similar helically coiled stent structures are known in the art.

US Publication No. 2004/267349 to Richter describes a class of medical devices and implants comprising amorphous metal alloys. The medical devices and implants may be temporary or permanent and may comprise other materials as well, such as polymers, ceramics, and conventional crystalline and polycrystalline metal alloys.

A problem in the art arises when trying to construct a stent from flat metal using new materials which may be stronger and more flexible, such as amorphous metal alloys. Because amorphous metals convert to an undesirable crystalline state upon welding, stents having a flat metal construction can not currently be manufactured with these materials.

One object of the invention relates to producing a stent having a flat metal construction without the need to weld the components together. Rather, in accordance with the invention the cylindrical form of the metal stent is maintained by a polymer layer.

Another object of the invention relates to a stent having a flat metal construction which is corrosion resistant, highly biocompatible and durable enough to withstand repeated elastic deformation, which are properties of an amorphous metal alloy stent made without the need to weld any part of the stent.

### SUMMARY OF THE INVENTION

The invention is defined by claims 1 and 10. Therefore, in accordance with these objects, the hybrid stent of the present invention comprises a first component and a second component, wherein the first component is a tubular structure having an amorphous metal alloy that is arranged in a helically coiled pattern. The second component is a biocompatible non-metallic material that maintains the tubular structure of the first component. The present invention includes a biocompatible non-metallic material that is wrapped around a tubular structure, as well as alternatively a biocompatible non-metallic material that is embedded into a tubular structure. The present invention further includes a method of making this hybrid stent by rolling an amorphous metal alloy strip into a helically coiled tubular structure and wrapping at least a portion of that tubular structure in a biocompatible non-metallic material, which maintains the helical tubular structure of the metal strip.

The present invention provides a stent that is longitudinally flexible such that it can easily be tracked down tortuous lumens and does not significantly change the compliance of the vessel after deployment, wherein the stent is relatively stable so that it avoids bending or tilting in a manner that would potentially obstruct the lumen and so that it avoids leaving significant portions of the vessel wall unsupported.

The present invention relates to an intraluminal prosthetic device containing at least one amorphous metal alloy. Such medical devices provide the advantage of corrosion resistance, resistance to unwanted permanent deformation, and radiation protection. Many medical devices can benefit from such enhanced physical and chemical properties. This invention contemplates intraluminal prosthetic devices comprising at least one amorphous metal alloy combined with components made of other materials, with biocompatible materials being particularly preferred. The medical devices may contain one or more
amorphous metal alloys. Such alloys provide improved tensile strength, elastic deformation properties, and reduced corrosion potential to the devices.

Amorphous metal stents are prepared from a flat metal. The stent components are in the form of strips. The strips are helically wound to produce a tubular structure which can function to hold open a blood vessel upon expansion. Generally, the instant invention can be made from any stent formed as a continuous elongated helical element preferably having spaced undulating portions forming periodic loop portions. In one embodiment, the stent may be formed of a strip helically wound into a series of coiled windings, wherein the strip is formed of at least two side bands connected to each other, for example, by a series of cross struts. Each side band is formed in a serpentine pattern comprising a series of bends, wherein upon expansion of the stent, the bends of the side bands open to increase the length of each of the individual cells in the helical direction, thereby lengthening the strip in the helical direction to allow the stent to expand without any significant unwinding of the strip. Because amorphous metal alloys cannot be easily welded without the metal reverting to an undesirable crystalline form, the present invention contemplates wrapping the helically wound amorphous metal alloy stent in a biocompatible non-metalic material, such as a polymer thereby forming a hybrid stent. Biocompatible materials include those materials considered to be biodegradable and/or bioresorbable as well as durable polymers.

The stent may be of any desired design. The stent may be made for implanting by either balloon expansion or self expansion.

### BRIEF DESCRIPTION OF THE DRAWINGS

Figure 1 illustrates a photomicrograph of stent members connected by a porous polymeric structure.
Figure 2 illustrates stent components in the form of a helical strip connected by a porous polymeric structure.
Figure 3 illustrates a stent element connected by a porous polymeric structure.

### DETAILED DESCRIPTION OF THE INVENTION

Amorphous metal alloys, also known as metallic glasses, are disordered metal alloys that do not have long-range crystal structure. Many different amorphous metal alloy compositions are known, including binary, ternary, quaternary, and even quinary alloys. Amorphous metal alloys and their properties have been the subject of numerous reviews (see for example, Amorphous Metal Alloys, edited by F.E. Luborsky, Butterworth & Co, 1983, and references therein).

Amorphous metal alloys have been used in the past primarily for items such as computer-related parts, golf club heads, and drill bit coatings. All these are articles made by the so-called bulk process. However, the present invention has recognized that amorphous metal alloys made in a continuous hot extrusion process, as described herein, possess physical and chemical properties which make them attractive candidates for use in medical devices. For example, amorphous metal alloys may have a tensile strength that is up to ten-fold higher than that of their conventional crystalline or polycrystalline metal counterparts. Also, amorphous metal alloys may have a ten-fold wider elastic range, *i.e.,* range of local strain before permanent deformation occurs. These are important features in medical devices to provide an extended fatigue-resistant lifespan for devices that are subjected to repeated deformations in the body. In addition, these features allow production of smaller or thinner devices that are as strong as their bulkier conventional counterparts.

Amorphous metal alloys exhibit significantly different physical properties compared to normal metals, owing to their disordered local microstructure. In contrast to normal metals, which typically contain defects such as grain boundaries and cavities, amorphous metal alloys typically exhibit a uniform random phase on a microscopic scale, and do not contain such defects. As a result, amorphous metal alloys do not experience the strains associated with grain boundaries and/or cavities, and therefore show superior mechanical properties, such as a high elastic modulus, high tensile strength, hardness, and fatigue resistance. Additionally, many studies have indicated that amorphous metal alloy have superior corrosion resistance compared to their crystalline counterparts. (See Amorphous Metal Alloys, edited by F.E. Luborsky, Butterworth & Co, 1983, p. 479). In particular, some amorphous metal alloys are known to resist corrosion even by anodic polarization in strongly acidic solutions (*e*.*g*., 12 M HCl).

This invention provides a new class of medical devices, in particular, stents comprising amorphous metal alloys manufactured by heat extrusion. The amorphous metal alloys contemplated by this invention possess the advantages of almost any desired alloy combination, no toxic additives, and corrosion resistance that results in drastic improvement in bio-compatibility. These amorphous metal alloys have many properties that make them suitable for use as implants, including high mechanical strength, resistance to fatigue, corrosion resistance, and biocompatibility. The stents of this invention may be implanted in animals, non-limiting examples of which include reptiles, birds, and mammals, with humans being particularly preferred. Besides containing at least one amorphous metal alloy, the implants of this invention may optionally contain other materials, including different types of amorphous metal alloys, conventional crystalline or polycrystalline metals or metal alloys, polymers, ceramics, and natural and synthetic biocompatible materials.

The devices may contain one or more amorphous metal alloys. The method of heat extrusion is very flexible and many combinations of metals can be made into an amorphous metal alloy. By way of example, iron-based, cobalt-based alloys, copper-based amorphous metal alloys, as well as others may be manufactured using heat extrusion as described herein (see Example 1). In certain embodiments, the amorphous metal alloys may comprise a metalloid, non-limiting examples of which include silicon, boron, and phosphorus. One possible amorphous metal alloy is an Fe-Cr-B-P alloy. Many other similar alloys are suitable and known to one of ordinary skill in the art.

In certain preferred embodiments, the amorphous metal alloys contemplated by this invention exhibit significantly lower conductance or are non-conductive, compared to their crystalline or polycrystalline counterparts.

The amorphous metal alloy components of this invention may be combined or assembled with other components, either amorphous metal or otherwise, in order to form intraluminal implants. For example, the amorphous metal alloy components may be combined with a biocompatible polymer, a biodegradable polymer, a therapeutic agent (e.g., a healing promoter as described herein) or another metal or metal alloy article (having either a crystalline or amorphous microstructure).

In particular, the stents of the present invention may be formed from flat metal which is rolled to form a tubular structure. The tubular structure is held in this position without the need for welding the ends by a second component, which wraps around the rolled amorphous metal tubular structure or is embedded into the metal structure. This second component may be a biodegradable or bioresorbable material which holds the amorphous metal alloy in its tubular structure for positioning and expansion in the lumen but is degraded after the stent is embedded in the vessel wall tissue. Alternatively, a durable biocompatible polymer may be employed as a second component in a similar manner.

The method of combining or joining the amorphous metal alloy components to other components can be achieved using methods that are well known in the art. Non-limiting examples of joining methods including physical joining (*e*.*g*., braiding, weaving, crimping, tying, and press-fitting) and joining by adhesive methods (*e*.*g*., gluing, dip coating, and spray coating). Combinations of these methods are also contemplated by this invention.

When a stent is implanted in a body lumen, such as an artery, with the stent having an initial diameter D₁, the stent can be flexed and bent easily in a meandering lumen during delivery. Then, the stent is expanded to have a second diameter D₂ which is larger than the initial diameter D₁ whereby the stent is implanted.

When the stent is delivered and expanded, a delivery catheter assembly with an expandable member, such as a balloon, may be used as is known in the art. When the catheter assembly with a balloon is used to deliver the stent, the stent is mounted on the balloon and the catheter assembly is pushed into the implantation site. Then, the balloon is inflated, radially applying a force inside the stent and the stent is expanded to its expanded diameter. Alternatively, the stent may be self-expanding in which case a balloon is not needed to facilitate expansion of the stent.

The implants of this invention may be temporary or permanent medical implants and comprise at least one amorphous metal alloy component. As used herein, an "implant" refers to an article or device that is placed entirely or partially into an animal, for example by a surgical procedure or minimally invasive methods. Many different types of implants may be formed of or contain amorphous metal alloys. Non-limiting examples include grafts, surgical valves, joints, threads, fabrics, fasteners, sutures, stents and the like. This invention contemplates intraluminal devices that comprise an amorphous metal alloy component (or components) combined with components made of other materials, with biocompatible materials being preferred.

A biocompatible material, as the term is used herein, is bioresorbable and/or biodegradable. Such a material is absorbed into or degraded by the body by active or passive processes. Similarly, certain biocompatible materials are "resorbed" by the body, that is, these materials are readily colonized by living cells so that they become a permanent part of the body. Such materials are also referred to herein as bioresorbable or durable polymers When either type of material is referred to anywhere in this application, it is meant to apply to both bioresorbable and biodegradable materials.

It is desirable to design the longitudinal structure of the stent so that it would promote the growth of neo-intima that will fix the amorphous metal alloy stent to the desired position before the longitudinal structure is absorbed or degraded, and thus prevent movement of the stent thereafter.

The longitudinal structure of the bioresorbable material may be porous or it may be formed as a tube with fenestrations or a series of fibers with spaces between them, to promote faster growth of neo-intima that will cover the stent and secure it in position before degradation of the material. Fenestrations may also promote better stabilization of the stent before degradation of the bioresorbable material. The shape of fenestration can be made in any desired size, shape or quantity.

It will be appreciated that the amorphous metal alloy stent's release from the biocompatible material is optional and can be controlled by the characteristics of the material chosen. Preferably, release occurs after the stent is buried in the neo-intima and the stent is stabilized.

The present invention allows the bioresorbable material to be manufactured at any length. In one embodiment, the stent in the supporting structure may be manufactured as a long tube and then cut to customize the length of the implanted stent for a particular patient.

Any stent design may be utilized with the bioresorbable or durable biocompatible polymer material in the manner taught by the present invention. In one example, sections of the helical strip can be any structure which provides a stored length to allow radial expansion. However, it should be understood that the invention is not limited to any particular helical ring structure or design. For example, the helical strip can be of the same design throughout the stent or the strip may be of different designs along its length depending on their intended use or deployment. Thus, the invention also permits a stent design in which various sections of the helical strip can have different structural or other characteristics to vary certain desired properties over the length of the stent. For example, the end sections of the strip can be made to produce more rigid (e.g., after expansion) stent sections than those in the middle of the stent.

This example is only given as an illustration and is not meant to limit the scope of the invention. Any stent design can be used in the present invention. The individual design of the helical strip can be uniform or not, depending on the application for the resulting stent.

Upon deployment in a vessel to cover a long lesion, the polymer material holds the rolled flat metal stent structure together until a time when the stent is embedded in the vessel wall neo-intimal structure. The structure now can articulate, move, or flex as the vessel flexes or stretches, to allow natural movement of the vessel wall. Thus, the amorphous metal alloy stent of the invention bends according to the natural curvature of the vessel wall. The same flexibility can be achieved by use of a flexible durable polymer.

The release time of the bioresorbable material as the longitudinal structure of the stent can be controlled by the characteristics of the bioresorbable material. Preferably, the stent will have been buried in a layer of neointima stabilized before the bioresorbable material is resorbed.

There are several advantages of using bioresorbable material or durable biocompatible polymers. These materials function as a second component of the amorphous metal alloy hybrid stent and function to hold the rolled flat metal stent structure in a tubular configuration for implantation into the vessel until the stent is embedded in vessel wall.

Additionally, these materials do not obscure radiographs or MRI/CT scans, which allows for more accurate evaluation during the healing process. Another advantage of using these materials is that the continuous covering provided by the material after the stent is deployed in a vessel is believed to inhibit or decrease the risk of embolization. Another advantage is the prevention of "stent jail" phenomenon, or the complication of tracking into side branches covered by the stent.

The depletion of the bioresorbable material covering can be controlled by modification or choosing characteristics of the bioresorbable material to allow degradation or resorption at a time about when the structure is fixated in the vessel wall and embolization is no longer a risk. Examples of altering the biodegradable or bioresorbable material by modification or changing the material characteristics of the polymer are described below as to the extent and speed a material can degrade. It should be understood that these modifications and characteristics are merely examples and are not meant to limit the invention to such embodiments.

Bioresorbable material can be, but is not limited to, a bioresorbable durable polymer. For example, any bioresorbable polymer can be used with the present invention, such as polyesters, expanded polytetrafluoroethylene (ePTFE), polyanhydrides, polyorthoesters, polyphosphazenes, polyurethane, silicones, polyolefins, polyamides, polycaprolactams, polyimides, polyvinyl alcohols, acrylic polymers and copolymers, polyethers, celluiosics and any of their combinations in blends or as copolymers. The biodegradable material can be any material that readily degrades in the body and can be naturally metabolized. Usable biodegradable polymers can include polyglycolide, polylactide, polycaprolactone, polydioxanone, poly(lactide-co-glycolide), polyhydroxybutyrate, polyhydroxyvalerate, trimethylene carbonate, polyphosphoesters, polyphosphoester - urethane, polyaminoacids, polycyanoacrylates, biomolecules such as fibrin, fibrinogen, cellulose, starch, collagen and hyaluronic acid and any blends, mixtures and/or copolymers of the above polymers.

Synthetic condensation polymers, as compared to addition type polymers, are generally biodegradable to different extents depending on chain coupling. For example, the following types of polymers biodegrade to different extents: polyesters biodegrade to a greater extent than polyethers, polyethers biodegrade to a greater extent than polyamides, and polyamides biodegrade to a greater extent than polyurethanes. Morphology is also an important consideration for biodegradation. Amorphous polymers biodegrade better than crystalline polymers. Molecular weight of the polymer is also important. Generally, lower molecular weight polymers biodegrade better than higher molecular weight polymers. Also, hydrophilic polymers biodegrade faster than hydrophobic polymers. There are several different types of degradation that can occur in the environment. These include, but are not limited to, biodegradation, photodegradation, oxidation, and hydrolysis. Often, these terms are combined together and are called biodegradation. However, most chemists and biologists consider the above processes to be separate and distinct. Biodegradation alone involves enzymatically promoted break down of the polymer caused by living organisms.

Employment of a light and porous polymeric material may provide several advantages. For example, a fibrous material may be constructed so that the fibers provide a longitudinal structure thereby enhancing the overall flexibility of the stent device. Such a material may be applied to a tubular stent in a continuous or non-continuous manner depending upon the particular needs of the structure contemplated. The material may be any polymeric material, as described above. The polymeric material can form a porous fiber mesh that is a durable polymer. The longitudinal polymeric structure serves at least two functions. First, the longitudinal polymeric structure is more longitudinally flexible than a conventional metallic structure. Second, the polymeric material is a continuous structure with small inter-fiber distance and can be used as a matrix for eluting drug that would provide a more uniform elution bed.

As a further advantage of the invention, the bioresorbable structure may be embedded with drug that will inhibit or decrease cell proliferation or will reduce restenosis in any way. Examples of such drugs include for example rapamycin and paclitaxol and analogs thereof. In addition, the stent may be treated to have active or passive surface components such as drugs that will be advantageous for the longer time after the stent is exposed by bioresorption of the longitudinal structure.

The stent may also include fenestrations. Fenestrations can be any shape desired and can be uniformly designed such as the formation of a porous material for example, or individually designed. The non-continuous layered material can also be formed in other ways such as a collection of bioresorbable fibers connecting the structure. Fenestration of the bioresorbable cover may promote faster growth of neo-intima and stabilization of the structure before degradation of the bioresorbable material. The present invention allows the bioresorbable material to be manufactured at any length and then cut in any desired length for individual functioning stents to assist manufacturing the stent. For example, in the case of bioresorbable polymer tubing, the tubing can be extruded at any length and then cut to customize the stent, either by the manufacturer or by the user.

Example designs are described in, but not limited to, U.S. Patent No. 6,723,119. One example design is the NIRflex stent which is manufactured by Medinol, Ltd. This design criteria preferably results in a structure which provide longitudinal flexibility and radial support to the stented portion of the vessel. Helically oriented strips of NIRflex cells, for example, may be manufactured and rolled into tubular amorphous metal stent structures. The tubular structure is held in position by a biocompatible material coating around the outside of the rolled tubular structure.

Another example of a flat metal stent is described in US Patent Nos. 6,503,270 and 6,355,059. In this example, the flat metal stent design is configured as a coiled stent in which the coil is formed from a wound strip of cells wherein the sides of the cells are serpentine. Thus, the stent is made up of a strip helically wound into a series of coiled windings, wherein the strip is formed of at least two side bands connected to each other, for example by a series of cross struts. In one embodiment, each side band of the strip is formed in a serpentine pattern comprising a series of bends, wherein upon expansion of the stent, the bends of the side bands open to increase the length of each of the individual cells in the helical direction, thereby lengthening the strip in the helical direction to allow the stent to expand without any significant unwinding of the strip. The two ends of the strip at the ends of the stent are joined, for example by welding to the respective adjacent windings, thereby creating smooth ends and assuring no relative rotation. This design retains the flexibility associated with coiled spring stents, yet has windings which are relatively stable and insusceptible to displacement or tilt. A serpentine coiled ladder stent thus provides continuous support of the vessel tissue without disadvantageously obstructing the lumen.

In one embodiment of the serpentine ladder design, the stent is configured as a coiled stent in which the coil is formed from a wound strip of cells wherein the side of the cells are serpentine.

Optionally, the ends of the helical strip may be tapered. The tapering of the ends of the strip allows the ends of the finished stent to be straight; i.e., it allows the stent to take the form of a right cylinder, with each of the ends of the cylindrical stent lying in a plane perpendicular to the longitudinal axis of the stent. These ends need not be welded but rather are wrapped with a biocompatible material.

The bioresorbable material can be disposed within interstices and/or embedded throughout the stent. The bioresorbable material may cover the entire exterior or only a portion of the stent structure or fully envelop the entire stent.

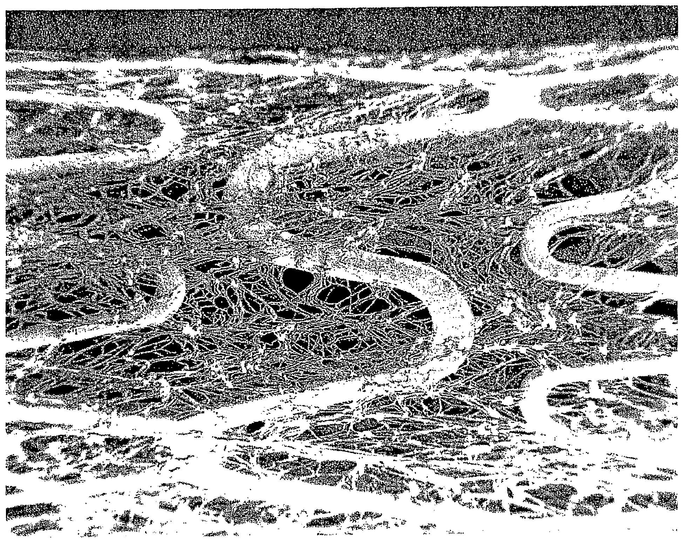

FIG. 1 shows a photomicrograph of an exemplary stent illustrating stent members connected by a biocompatible material, which includes, but is not limited to, a polymeric porous structure. The stent of Figure 1 is connected by a porous longitudinal structure along a longitudinal axis of the stent. This longitudinal structure may or may not be polymeric, depending on the properties desired. In one embodiment, the longitudinal structure is a porous fiber mesh like a durable polymer. One example of such a material includes, but is not limited to, polytetrafluoroethylene (ePTFE). The longitudinal structure, among other functions, provides longitudinal flexibility to the stent structure. The stent is preferably an amorphous metal alloy structure. The longitudinal structure provides a continuous structure having small inter-fiber distances and forming a matrix. This matrix may be used for eluting a drug and provides a more uniform elution bed over conventional methods.

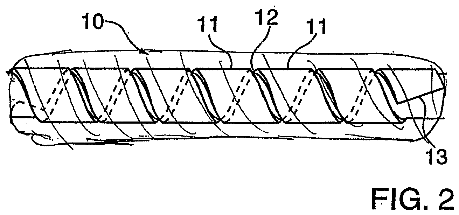

FIG. 2 shows an example coiled ribbon stent 10 disposed in a porous fiber mesh 12. As shown in FIG. 2, the coiled ribbon stent is formed as a helically wound ribbon strip having ends 13 and windings 11. Depending on the embodiment, the windings 11 of the coiled ribbon stent 10 are relatively resistant to longitudinal displacement or tilting because of the width of the ribbon in the coiled ribbon stent 10. The mesh 12, although allowing longitudinal flexibility of the stent, further provides support to the stent to resist longitudinal displacement or tilting.

Expansion of the coiled ribbon stent 10 of FIG. 2 may be accomplished, for example, by inflating a balloon on a catheter (not shown). The outward force of the balloon acts on the inside of the stent 10 causing the stent 10 to expand. When the coiled ribbon stent 10 is expanded, the diameter of the individual windings 11 increases. However, because the length of the ribbon strip is constant, the increase in diameter may cause the ribbon strip to unwind somewhat, in order to accommodate the expansion. In doing so, the ends 13 of the stent 10 rotate, the number of windings 11 decreases, and the overall length of the stent foreshortens and/or gaps are formed between adjacent windings 11. The porous fiber mesh 12 that is disposed about the coiled ribbon stent 10 provides protection of the rotation of the stent, particularly of the stent ends, that may be potentially harmful to the vessel.

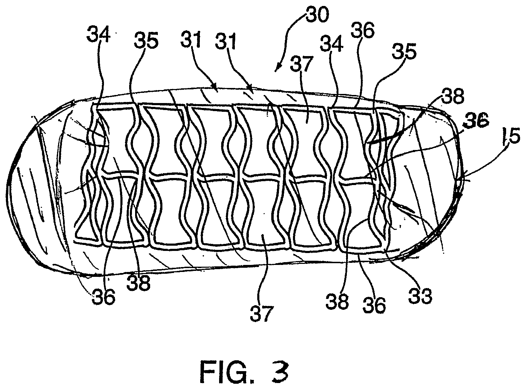

In addition, the porous fiber mesh 12 also provides coverage between gaps in the windings of the coiled ribbon stent 10. The porous fiber mesh may assist in providing some support between these gaps. FIG. 3 shows a serpentine coiled ladder stent 30 constructed in accordance with the invention. The serpentine coiled ladder stent 30 in FIG. 3 is shown having a porous fiber mesh 15 disposed about the stent.

The serpentine coiled ladder stent 30 illustrated in FIG. 3 is configured as a coiled stent in which the coil is formed from a wound strip of cells 37, wherein the sides of the cells 37 are serpentine. The stent in this illustration is comprised of a strip helically wound into a series of coiled windings 31, wherein the strip is formed of two side bands 34, 35 connected to each other, for example by a series of cross struts 36. Each side band 34, 35 is formed in a serpentine pattern comprising a series of bends 38. Upon expansion of the stent, the bends 38 of the side bands 34, 35 open to increase the length of each of the individual cells 37 in the helical direction. Thus, lengthening the strip in the helical direction is permitted for the stent 30 so the stent may expand without any significant unwinding of the strip, or foreshortening.

In this illustrated embodiment of FIG. 3, the bends in the side bands 34, 35 occur in a periodic pattern. The bends 38 may be arranged, for example, in the pattern of a sine wave, or in any other suitable configuration.

Depending on the embodiment, the stent may be described as a series of square cells 37 or triangular cells. The side bands 34, 35 and the cross struts 36 form the perimeter of each cell. In the unexpanded state, the side bands are collapsed to form a serpentine continuum.

In the illustrated embodiment of FIG. 3, the cross struts 36 joining the side bands 34, 35 to each other are straight and extend in a direction generally perpendicular to the helical direction in which the strip is wound. Alternatively, the cross struts may have one or more bends, and/or they may extend between the two side bands at other angles. In the illustrated embodiment, the cross struts 36 join oppositely facing bends 38 on the side bands 34, 35, and they are attached to the side bands 34, 35 at every second bend 38. Alternatively, the cross struts 36 may be joined in other places, and may occur with more or less frequency, without departing from the general concept of the invention. The stent alternatively may be made without cross struts 36, by having the two serpentine side bands 34, 35 periodically joined to each other at adjacent points.

Furthermore, as shown in FIG. 3, the ends 33 of the serpentine ladder strip may be tapered. The tapering of the ends 33 of the strip allows the ends of finished stent to be straight, i.e., it allows the stent to take the form of a right cylinder, with each of the ends of the cylindrical stent lying in a plane perpendicular to the longitudinal axis of the stent. The ends 33 of the strip if made from an amorphous metal may not be easily joined, for example by welds, to respective adjacent windings 31. In one example, the porous fiber mesh 15 may be used in this situation to join ends 33 to respective adjacent windings 31.

Below are further examples of various embodiments of the invention. Accordingly, it is to be understood that the present invention is described by way of example, and not by limitation.

### EXAMPLE 1: Methods of making amorphous metal alloys

Many different methods may be employed to form amorphous metal alloys. A preferred method of producing medical devices according to the present invention uses a process generally known as heat extrusion, with the typical product being a continuous article such as a wire or a strip. The process does not involve additives commonly used in the bulk process that can render the amorphous metal alloy non-biocompatible and even toxic. Thus, the process can produce highly biocompatible materials. In preferred embodiments, the continuous amorphous metal alloy articles are fabricated by a type of heat extrusion known in the art as chill block melt spinning. Two common chill block melt spinning techniques that produce amorphous metal alloy articles suitable for the medical devices of the present invention are free jet melt-spinning and planar flow casting. In the free jet process, molten alloy is ejected under gas pressure from a nozzle to form a free melt jet that impinges on a substrate surface. In the planar flow method, the melt ejection crucible is held close to a moving substrate surface, which causes the melt to be simultaneously in contact with the nozzle and the moving substrate. This entrained melt flow dampens perturbations of the melt stream and thereby improves ribbon uniformity. (See *e*.*g*., Liebermann, H. et al., "Technology of Amorphous Alloys" Chemtech, June 1987). Appropriate substrate surfaces for these techniques include the insides of drums or wheels, the outside of wheels, between twin rollers, and on belts, as is well known in the art.

Suitable planar flow casting and free-jet melt spinning methods for producing amorphous metal alloy components for the medical devices of this invention are described in U.S. Patent Nos. 4,142,571; 4,281,706; 4,489,773, and 5,381,856; For example, the planar flow casting process may comprise the steps of heating an alloy in a reservoir to a temperature 50-100 °C above its melting temperature to form a molten alloy, forcing the molten alloy through an orifice by pressurizing the reservoir to a pressure of about 3.4 kPa - 13.8 kPa (0.5-2 PSI) and impinging the molten alloy onto a chill substrate, wherein the surface of the chill substrate moves past the orifice at a speed of between 300 - 1600 meters/minute and is located between 0.03 to 1 millimeter from the orifice. In embodiments involving free-jet melt spinning, the process may comprise the steps of heating an alloy in a reservoir to a temperature above the melting point of the alloy, ejecting the molten alloy through an orifice in the reservoir to form a melt stream with a velocity between 1-10 meters/second, and impinging the melt stream onto a chill substrate, wherein a surface of the chill substrate moves past the orifice at a speed of between 12 - 50 meters/second.

Besides quenching molten metal (e.g., chill block melt spinning), amorphous metal alloys can be formed by sputter-depositing metals onto a substrate, ion-implantation, and solid-phase reaction. Each of these methods has its advantages and disadvantages. The choice of a particular method of fabrication depends on many variables, such as process compatibility and desired end use of the amorphous metal alloy article.

In some embodiments of the invention, amorphous metal alloy components for implants may be used, *i.e.* parts of the implant are made of amorphous metal alloys. These parts may be provided in a variety of ways. For example, the component may be produced by machining or processing amorphous metal alloy stock (*e*.*g*., a wire, ribbon, rod, tube, disk, and the like). Amorphous metal alloy stock made by chill block melt spinning can be used for such purposes.

It should be understood that the above description is only representative of illustrative examples of embodiments. For the reader's convenience, the above description has focused on a representative sample of possible embodiments, a sample that teaches the principles of the invention. Other embodiments may result from a different combination of portions of different embodiments. The description has not attempted to exhaustively enumerate all possible variations.

Again, the embodiments described herein are examples only, as other variations are within the scope of the invention as defined by the appended claims.

## Claims

1. A stent (10) comprising a helically coiled flat pattern (11) made of metal having an amorphous metal alloy composition and a biocompatible material layer (12) around the coiled amorphous metal alloy composition
**characterized by**
said helically coiled flat pattern (11) being in the form of a strip, wherein the strip is helically wound to produce a tubular structure, said helically coiled flat pattern (11) being held by said biocompatible material layer (12) wrapped around the tubular structure or embedded into the tubular structure.

2. The stent (10) according to claim 1, wherein the helically coiled flat pattern (11) has a first side band connected to a second side band, each of the first and second side bands formed in a serpentine pattern having a series of bends.

3. The stent (10) according to claim 1, wherein the biocompatible material layer (12) is a porous material.

4. The stent (10) according to claim 1, wherein the biocompatible material layer (12) is durable.

5. The stent (10) according to claim 1, wherein the biocompatible material layer (12) is expanded polytetrafluoroethylene (ePTFE).

6. The stent (10) according to claim 1 wherein the amorphous metal alloy comprises an Fe-Cr-B-P alloy.

7. The stent (10) according to claim 1, wherein the amorphous metal alloy contains silicon.

8. The stent (10) according to claim 1 further comprising a drug coating.

9. The stent (10) according to claim 1, wherein the biocompatible material layer (12) is a fiber mesh.

10. A method of making a stent (10) according to any of claims 1 to 9 comprising
rolling a flat metal strip (11) having a serpentine pattern into a tubular structure, wherein the flat metal strip (11) comprises at least one amorphous metal alloy; and
covering at least a portion of the tubular structure with a biocompatible material.

11. The method of claim 10, wherein the biocompatible material is expanded polytetrafluoroetlyene (ePTFE).

12. The method of claim 10, wherein the stent (10) is a coiled strip having cells.

13. The method of claim 12, wherein the cells have side walls that are serpentine.

## Patentansprüche

1. Stent (10), aufweisend ein helixförmig gewickeltes Flachmuster (11), das aus Metall besteht, welches eine amorphe Metalllegierungskomposition und eine Schicht aus biokompatiblem Material (12) um die gewickelte amorphe Metalllegierungskomposition aufweist,
**dadurch gekennzeichnet, dass**
das helixförmig gewickelte Flachmuster (11) die Form eines Streifens aufweist, wobei der Streifen helixförmig gewickelt ist, um eine röhrenförmige Struktur zu erzeugen, wobei das helixförmig gewickelte Flachmuster (11) durch die Schicht aus biokompatiblem Material (12) gehalten wird, die um die röhrenförmige Struktur gewickelt ist oder in die röhrenförmige Struktur eingebettet ist.

2. Stent (10) nach Anspruch 1, wobei das helixförmig gewickelte Flachmuster (11) ein erstes Seitenband aufweist, das mit einem zweiten Seitenband verbunden ist, wobei sowohl das erste als auch das zweite Seitenband in einem Serpentinenmuster mit einer Reihe an Biegungen ausgebildet ist.

3. Stent (10) nach Anspruch 1, wobei die Schicht aus biokompatiblem Material (12) ein poröses Material ist.

4. Stent (10) nach Anspruch 1, wobei die Schicht aus biokompatiblem Material (12) langlebig ist.

5. Stent (10) nach Anspruch 1, wobei die Schicht aus biokompatiblem Material (12) expandiertes Polytetrafluorethylen (ePTFE) ist.

6. Stent (10) nach Anspruch 1, wobei die amorphe Metalllegierung eine Fe-Cr-B-P-Legierung aufweist.

7. Stent (10) nach Anspruch 1, wobei die amorphe Metalllegierung Silizium enthält.

8. Stent (10) nach Anspruch 1, ferner aufweisend eine Arzneimittelbeschichtung.

9. Stent (10) nach Anspruch 1, wobei die Schicht aus biokompatiblem Material (12) ein Fasergeflecht ist.

10. Verfahren zum Herstellen eines Stents (10) nach einem der Ansprüche 1 bis 9, aufweisend:
Walzen eines flachen Metallstreifens (11) mit einem Serpentinenmuster in eine röhrenförmige Struktur, wobei der flache Metallstreifen (11) zumindest eine amorphe Metalllegierung aufweist; und
Bedecken zumindest eines Abschnitts der röhrenförmigen Struktur mit einem biokompatiblen Material.

11. Verfahren nach Anspruch 10, wobei das biokompatible Material expandiertes Polytetrafluorethylen (ePTFE) ist.

12. Verfahren nach Anspruch 10, wobei der Stent (10) ein gewickelter Streifen mit Zellen ist.

13. Verfahren nach Anspruch 12, wobei die Zellen Seitenwände aufweisen, die serpentinenförmig sind.

## Revendications

1. Stent (10) comprenant un motif plat enroulé de façon hélicoïdale (11) constitué d'un métal ayant une composition d'alliage métallique amorphe et une couche de matériau biocompatible (12) autour de la composition d'alliage métallique amorphe enroulée
**caractérisé par**
ledit motif plat enroulé de façon hélicoïdale (11) étant sous la forme d'une bande, dans lequel la bande est enroulée de façon hélicoïdale afin de produire une structure tubulaire, ledit motif plat enroulé de façon hélicoïdale (11) étant maintenu par ladite couche de matériau biocompatible (12) enroulée autour de la structure tubulaire ou intégrée dans la structure tubulaire.

2. Stent (10) selon la revendication 1, dans lequel le motif plat enroulé de façon hélicoïdale (11) possède une première bande latérale reliée à une seconde bande latérale, chacune des première et seconde bandes latérales étant formée dans un motif de serpentin ayant une série de coudes.

3. Stent (10) selon la revendication 1, dans lequel la couche de matériau biocompatible (12) est un matériau poreux.

4. Stent (10) selon la revendication 1, dans lequel la couche de matériau biocompatible (12) est durable.

5. Stent (10) selon la revendication 1, dans lequel la couche de matériau biocompatible (12) est du polytétrafluoroéthylène expansé (ePTFE).

6. Stent (10) selon la revendication 1, dans lequel l'alliage métallique amorphe comprend un alliage Fe-Cr-B-P.

7. Stent (10) selon la revendication 1, dans lequel l'alliage métallique amorphe contient du silicium.

8. Stent (10) selon la revendication 1 comprenant en outre un enrobage de médicament.

9. Stent (10) selon la revendication 1, dans lequel la couche de matériau biocompatible (12) est un maillage de fibres.

10. Procédé de fabrication d'un stent (10) selon l'une quelconque des revendications 1 à 9 comprenant
l'enroulement d'une bande métallique plate (11) ayant un motif de serpentin en une structure tubulaire, dans laquelle la bande métallique plate (11) comprend au moins un alliage métallique amorphe ; et
le recouvrement d'au moins une partie de la structure tubulaire avec un matériau biocompatible.

11. Procédé selon la revendication 10, dans lequel le matériau biocompatible est du polytétrafluoroéthylène expansé (ePTFE).

12. Procédé selon la revendication 10, dans lequel le stent (10) est une bande enroulée ayant des cellules.

13. Procédé selon la revendication 12, dans lequel les cellules ont des parois latérales qui sont en serpentin.
